# EUROPEAN PATENT APPLICATION

(11) **EP 0 832 689 A2**
(43) Date of publication of application: **01.04.1998**
(21) Application number: 97115413.3
(22) Date of filing: 05.09.1997
(51) Int. Cl.: B01L 3/00, A61B 10/00

(54) **Specimen collection kit**

(30) Priority: 30.09.1996 US 719833
(71) Applicant: Becton, Dickinson and Company, Franklin Lakes, New Jersey 07417-1880 (US)
(72) Inventor: Bennett, Michael C., Summit, New Jersey (US)
(74) Representative: Selting, Günther, Dipl.-Ing.

(57) **Abstract**

A kit of parts for collecting and transporting specimens. The kit includes a first container to accommodate a plurality of components for collecting a specimen and a second container is adapted to nestably accommodate the first container. The first container is also used to accommodate used components subsequent to the collection procedure and the second container is used to transport the collected specimen.

## Description

### BACKGROUND OF THE INVENTION

### 1. Field of the Invention

This invention relates to a kit for use to obtain a specimen and more particularly, to a kit for use to obtain a specimen, to subsequently ship the specimen to a test facility and to safely contain and dispose of instruments used in the collecting of the specimen.

### 2. Description of Related Art

The collection of specimens for diagnostic testing is typically accomplished in a health care facility such as a hospital or laboratory. These facilities are suitably equipped and employ trained personnel so as to safely and efficiently conduct a specimen collection procedure. Furthermore, these facilities also provide for the safe disposal of the instruments used to collect the specimen. Such specimens may include various biological fluids such as blood or urine. Particularly, with respect to the collection of blood specimens, phlebotomists use blood collection tubes to collect the blood for subsequent testing. Various instruments, such as hypodermic needles, are also employed to take the blood specimen from the patient. The hospital or health care facility provides containers or receptacles for the safe disposal of the needles used to obtain the blood specimens.

Outside the professional health care facility it is also necessary to obtain specimens from a patient. In situations such as home health care, nursing homes, and assisted living facilities, a patient's specimen such as a blood specimen must also be obtained. Often in the home health care setting or in other non-hospital settings, the instruments and containers necessary for collection of specimens such as blood specimens may not be available.

Therefore, it is desirable to provide a system which may be used to safely obtain, collect and ship a specimen as well as dispose of the instruments used in the collecting of the specimen.

### SUMMARY OF THE INVENTION

The present invention is a kit comprising parts for collecting and transporting a specimen. The kit includes a collection receptacle to contain the specimen, a first container to accommodate the plurality of components and the collection receptacle and a second container to nestably accommodate the first container. The first container is adapted to accommodate the used components subsequent to the collection procedure. The second container also accommodates the collection receptacle subsequent to the collection procedure for shipment. Information pertaining to the patient and the specimen may be placed on the second container. Further shipping information may also be placed on the second container.

In using the kit of the present invention, the specimen collection instruments as well as the specimen collection receptacle are provided in a first container. The first container is nestably insertable into a second container. A specimen is collected in the collection receptacle employing the collection instruments. The collection instruments are sealably enclosed within the first container for disposal. The collection receptacle is sealably enclosed within the second container for subsequent shipment to a testing facility.

A notable advantage of the kit of the present invention is that it provides parts including containers, which accommodate the instruments used in conducting a specimen collection procedure and which provide for disposal of the instruments subsequent to the collection procedure.

In addition, the present invention provides a container that accommodates the receptacle used to contain the specimen for subsequent shipment to a testing facility.

Furthermore, the kit of the present invention provides a method for conducting a specimen collection procedure where a specimen can be obtained and the instruments used in collecting the specimen can be disposed of.

### DESCRIPTION OF THE DRAWINGS

FIG. 1 is an exploded perspective view of the components of the collection kit of the present invention including a first container which is nestably accommodated within a second container.

FIG. 2 is an exploded perspective view of the first container of FIG. 1 showing the contents thereof.

FIG. 3 is a flat plan view of the label employed over the second container of the kit of FIG. 1.

FIG. 4 is an exploded perspective view of the second container of FIG. 1 showing collection tubes accommodated within the container.

FIG. 5 is an exploded perspective view of the disposable instruments used in the collection kit of the present invention contained within the first collection container for disposal.

### DETAILED DESCRIPTION

While this invention is satisfied by embodiments in many different forms, there is shown in the drawings and will herein be described in detail, the preferred embodiments of the invention, with the understanding that the present disclosure is to be considered as exemplary of the principles of the invention and is not intended to limit the invention to the embodiments illustrated. Various other modifications will be apparent to and readily made by those skilled in the art without departing from the scope and spirit of the invention. The scope of the invention will be measured by the appended claims and their equivalents.

The present invention is directed to a kit of parts used in specimen collection procedures. Such procedures may include the collection of biological fluids such as blood or urine. Although the invention will be described with respect to obtaining a blood specimen, it may be appreciated that the concept of the present invention may be applied to the collection of other specimens and procedures where biohazardous materials is involved.

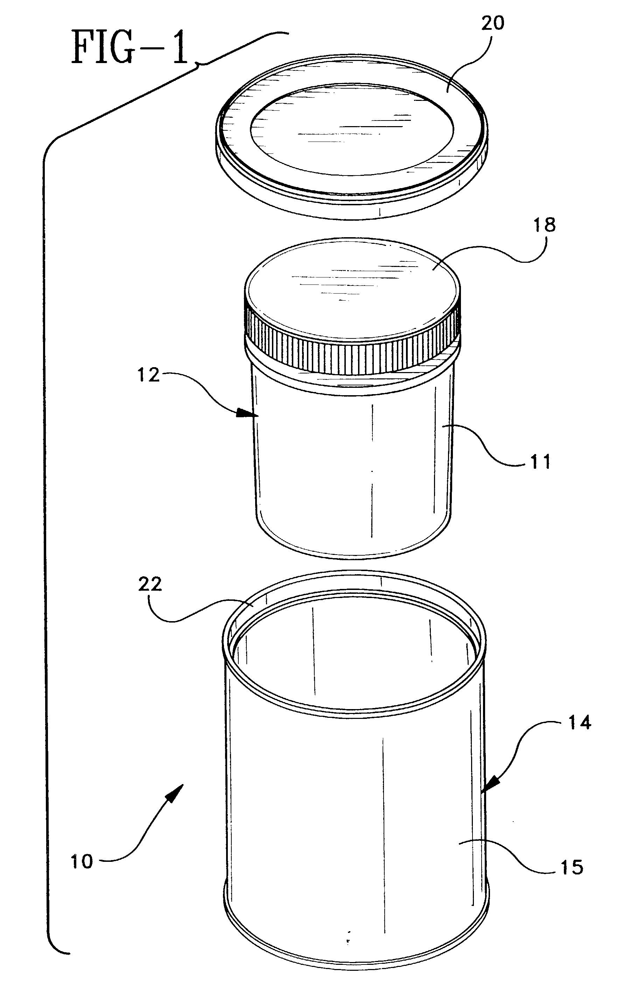

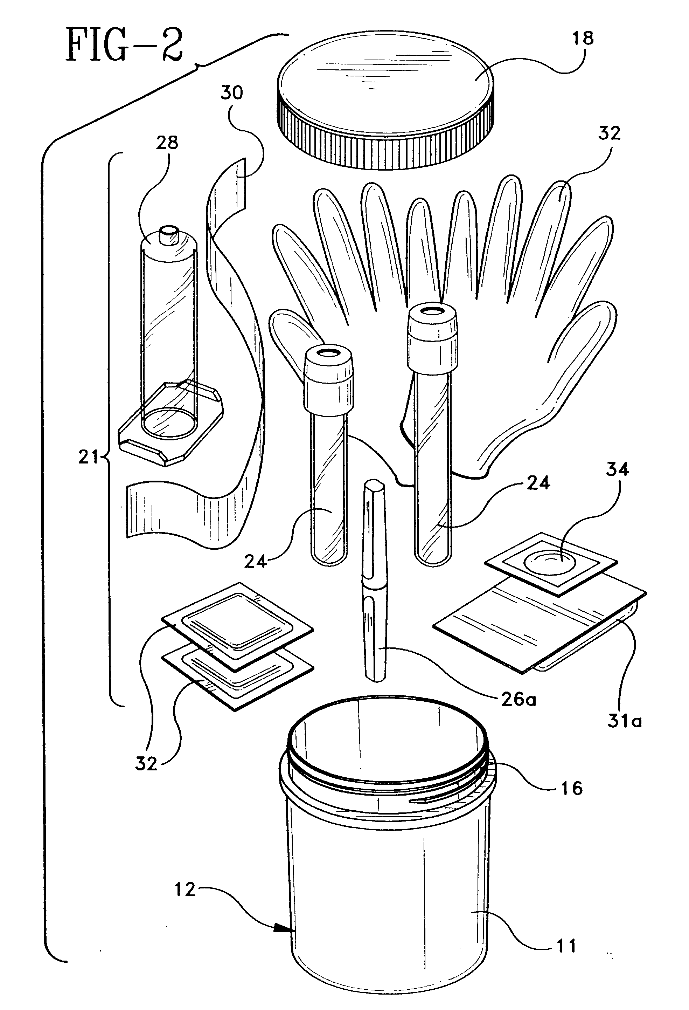

As shown in FIGS. 1 and 2, a blood collection kit **10** of includes a first container **12** which may be nestably accommodated within a second container **14**. First container **12** is generally an open-ended cylindrical member **11** formed of a suitable puncture resistant polymer such as polypropylene. First container **12** includes an internally threaded screw cap **18** which engages an externally threaded upper end **16** of cylindrical member **11** so as to provide sealing closure. First container and cap may also contain features to prevent the first container from being re-opened. First container **12** is designed to be snugly accommodated within second container **14** which also includes a generally open-ended cylindrical member **15**. Second container **14** may be formed of any suitably rigid material and most preferably is formed of a metal or cardboard. Second container **14** includes a cover **20** which may be disposed over the open upper end **22** of cylindrical member **15** to provide sealing closure of second container **14**.

As shown in FIG. 2, contents of first container **12** are typically used to obtain a patient's blood specimen. Accordingly, the contents typically used to collect blood are referred to as components **21**. It may be appreciated, however, that different components, depending upon the medical procedure conducted, may also be included within first container **12**.

In addition to components **21**, the contents of first container **12** may include one or more blood collection tubes **24**. These tubes may be any conventional blood collection tubes known in the art which are designed to contain a patient's blood sample. Such tubes include, but are not limited to coagulation tubes, glucose tubes, serum tubes, plasma tubes and the like.

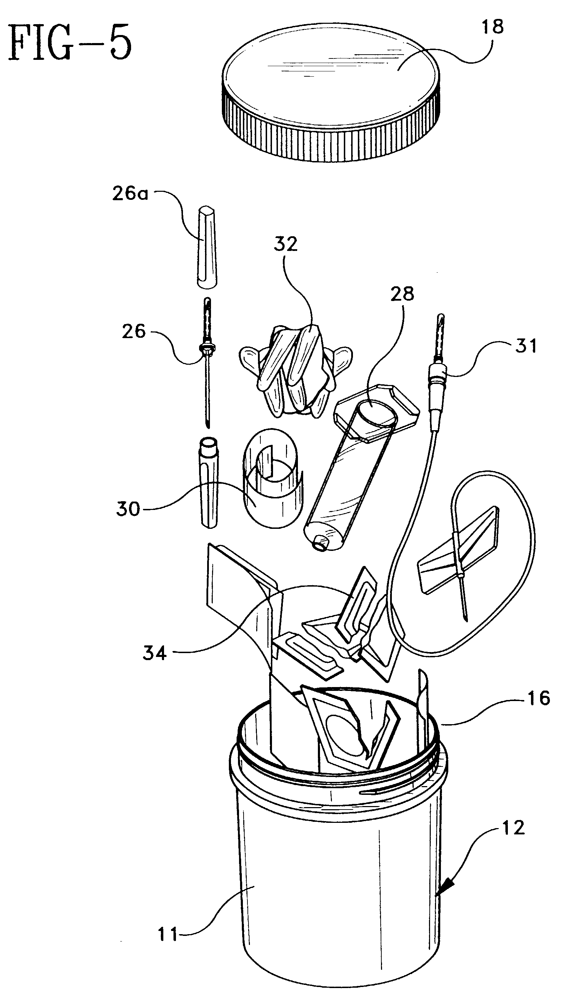

Components **21** typically used in blood collection may also include a blood collection hypodermic needle **26** (FIG. 5) which is enclosed in a protective covering **26a**. Needle **26** is used with a needle holder **28**, which may be employed in combination with blood collection tubes **24** to obtain the blood sample. A disposable tourniquet **30** is typically employed for elevation of venous pressure for collection of blood specimens. Further, disposable medical gloves **32** of the type worn by phlebotomists and other professionals for the collection of blood may also be included among components **21**. Additionally, various other instruments such as a package **31a** containing a blood collection set **31** (FIG. 5) for blood collection use, antiseptic wipes or swabs **32** for cleansing and sterilizing the site prior to puncturing the skin, and a bandage **34** for application over the needle puncture site after collection of the blood specimen may also form part of blood collection kit **10**. Various other components such as syringes, catheters, gauze, blood lancets, pre-filled syringes and the like may also be included.

Components **21** as well as blood collection tubes **24**, may all be contained within first container **12.** First container **12** may then be placed within second container **14** in nested fashion for distribution to the location where the blood sample is to be taken. Such locations may include nursing homes, physicians offices, private residences and the like where a patient's blood sample must be obtained.

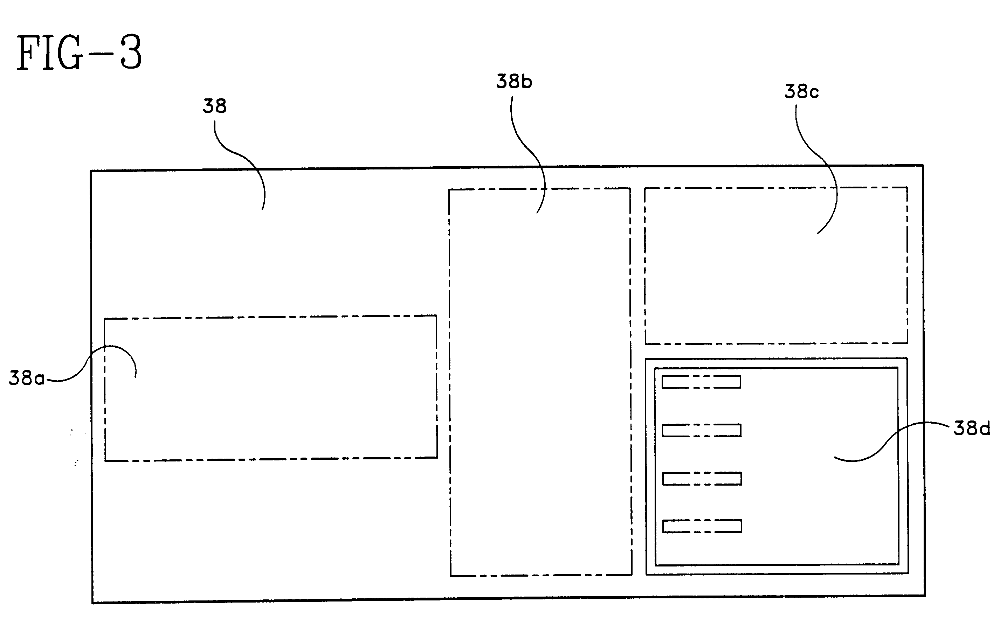

Second container **14** forms an outer package which allows kit **10** to be transported to the collection site. In that regard, second container **14** may include certain instructional information thereon. As shown in FIG. 3, second container **14** may include a label **38** which provides discrete areas for inclusion of various information. For example a first area **38a** may be provided for identification purposes. More specifically area **38a** may be used to identify second container **14** as being a kit for use in blood collection. A second area **38b** may list individually the contents included within kit **10**. A third area **38c** may provide instructions for the use of components **21** and blood collection tubes **24.** Furthermore, instructions such as shipping instructions for the shipping of the blood sample after collection, as will be described in further detail hereinbelow, may also be provided in area **38c**. A fourth area **38d** may be provided to accommodate patient supplied information. This information may include the patient's name, date of the procedure, type of tests which are to be conducted and the like. This area may be printed upon by the patient or the person conducting the test with suitable marking implements (not shown) so as to be permanently marked for shipment.

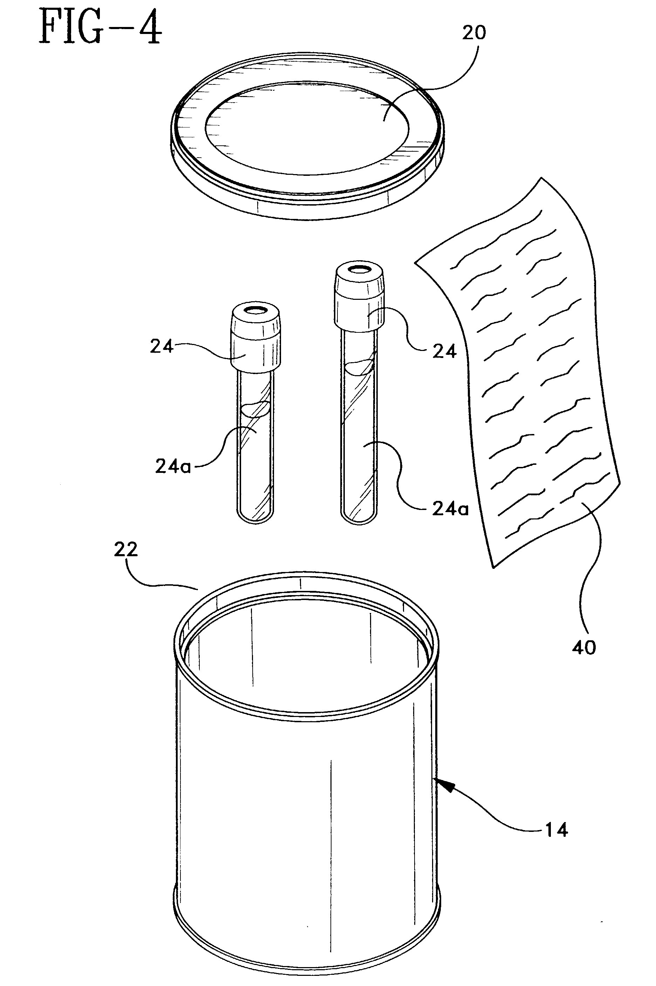

Having described the basic elements of blood collection kit **10** shown herein, its use and operation may be described with respect to the Figures shown herein. The blood collection kit **10** of the present invention is supplied with components **21** and blood collection tubes **24** enclosed within first container **12**. First container **12** is then enclosed within second container **14** for transport to the location where the blood collection procedure is to take place. Once at the blood collection location, the second container **14** is opened by removing cover **20**. First container **12** is then removed therefrom. The components **21** and tubes **24** are then removed from first container **12** so as to enable the phlebotomist to collect the blood sample. The blood collection procedure is conducted in a manner which is conventional and consistent with standard medical practices using the components **21** shown in FIG. 2. Once the blood specimen **24a** is collected, (FIG. 4) the blood collection tubes **24** may be reinserted directly into second container **14** which may then be closed by cover **20.** The second container **14** including the blood collection tubes **24** may then be transported to a test facility in accordance with shipping instructions contained on the label **38** of second container **14**. Prior to transporting such blood specimens, testing documentation such as forms **40** may also be inserted into second container **14**. These forms may include instructions as to the type of tests which are to be conducted as well as additional patient information. Also prior to transporting second container **14** enclosing the blood collection tubes **24**, additional patient information may be placed on label **38** at area **38b** so as to assure proper handling and testing of the blood collection tubes **24** contained therein.

Referring specifically to FIG. 5, certain of the components **21** used in the collection of the blood sample are designed for one time use and must be properly disposed of after the procedure. Such components including the blood collection needle **26**, the needle holder **28,** the tourniquet **30**, the medical gloves **31**, as well as debris such as the used antiseptic swabs **32**, wrappers **34a** from bandage **34** and the like, may be safely disposed of by inserting those components **21** into cylindrical member **11** of first container **12**. Cap **18** may be then screw threaded onto the upper end **16** to sealably enclose the used components therein. Cap **18** may be designed to interfit with screw threaded upper end **16** in a manner such that once the cap **18** is completely screw threaded, it is locked onto upper end **16** and cannot be reopened after closing. This prevents reaccess to the used components **21** further reducing the chance of a biomedical contamination. Furthermore, as first container **12** is formed of a suitably rigid puncture resistant polymer, the risk of exposing the contents of the container are substantially eliminated. The first container **12** is designed for suitable safe disposal such as by incineration. Thus, there is no need to empty the contents thereof after use. In addition, during shipment of first container **12** to a disposal site, an indication of the contents thereof is provided thereon by inclusion of information indicia in both graphic detail and written detail. Written information such as ACAUTION@, ABIOHAZARD@, AHYPODERMIC EQUIPMENT@ and the like, as well as graphic information such as a biohazard symbol may be included on the container to provide suitable information to all who may come in contact with first container **12**. Thus kit **10** of the present invention provides for the efficient method of obtaining of a blood collection sample, the shipping of the blood collection sample to the test facility, as well as the disposal of the used instruments employed in the blood collection procedure.

## Claims

1. A kit of parts for conducting a specimen collection procedure comprising:
a plurality of components used to obtain said specimen;
a collection receptacle used to retain said specimen;
a first container adapted to accommodate said plurality of components and said collection receptacle; and
a second container adapted to nestably accommodate said first container;
said first container being further adapted to accommodate said used components subsequent to said collection procedure;
said second container being further adapted to accommodate said collection receptacle subsequent to said collection procedure for shipment.

2. The kit of Claim 1 wherein said second container includes indicia thereon indicating shipping information.

3. The kit of Claim 1 wherein said first container includes indicia thereon indicating the contents.

4. The kit of Claim 2 wherein said second container includes user accessible areas for placement of patient and specimen information.

5. The kit of Claim 1 wherein said first container includes a removable cap for closing said first container.

6. The kit of Claim 4 wherein said second container includes a removable cover for closing said second container.

7. The kit of Claim 6 wherein said plurality of components includes a hypodermic needle and a collection tube.

8. The kit of Claim 7 wherein said first container is formed of puncture resistant material.

9. The kit of Claim 8 wherein said second container is adapted to accommodate said collection tube subsequent to said collection procedure.

10. A method of conducting a specimen collection procedure comprising the steps of:
providing specimen collection instruments for collecting said specimen;
providing a specimen collection receptacle for containing said specimen;
providing a first container accommodating said specimen collection instruments and said specimen collection receptacle;
providing a second container insertably accommodating said first container;
removing said first container from said second container;
removing said specimen collection instruments and said specimen collection receptacle from said first container;
collecting said specimen in said collection receptacle using said collection instruments;
sealably enclosing said used collection instruments within said first container for disposal; and
enclosing said specimen collection receptacle within said second container for subsequent shipment.
